Europäisches Patentamt

European Patent Office

Office européen des brevets

(11) Publication number: **0 513 354 A1**

# EUROPEAN PATENT APPLICATION
## published in accordance with Art. 158(3) EPC

(21) Application number: 91903828.1

(22) Date of filing: **18.02.91**

(86) International application number:
**PCT/JP91/00201**

(87) International publication number:
**WO 92/04420 (19.03.92 92/07)**

(51) Int. Cl.5: **C09K 15/34**, A23L 3/3472

(30) Priority: **31.08.90 JP 228305/90**
**28.11.90 JP 326826/90**
**30.11.90 JP 335712/90**

(43) Date of publication of application:
**19.11.92 Bulletin 92/47**

(84) Designated Contracting States:
**AT BE CH DE DK ES FR GB GR IT LI LU NL SE**

(71) Applicant: **DAINIPPON INK AND CHEMICALS, INC.**
**35-58, Sakashita 3-chome**
**Itabashi-ku, Tokyo 174(JP)**

(72) Inventor: **NISHIHASHI, Hideji**
**2-23-9, Oosakidai**
**Sakura-shi, Chiba 285(JP)**
Inventor: **KATABAMI, Tadashi**
**7-13-19, Maebaranishi**
**Funabashi-shi, Chiba 274(JP)**
Inventor: **MIYAGAWA, Kazuyuki**
**2-19-19, Asahigaoka**
**Yotsukaido-shi, Chiba 284(JP)**

(74) Representative: **Laurent, Michel et al**
**Cabinet LAURENT et CHARRAS, 20, rue Louis**
**Chirpaz B.P. 32**
**F-69131 Ecully Cedex(FR)**

(54) Antioxidizing composition and composition containing the same.

(57) An antioxidizing composition prepared by extracting sunflower seeds or residues of pressing thereof with water or an aqueous alcohol, and more particularly one wherein the aqueous alcohol is methanol and/or ethanol, one wherein the alcohol content of the aqueous alcohol is 90 vol % or less, and one wherein the alcohol content of the aqueous alcohol is 75 vol % or less; another antioxidizing composition prepared by hydrolyzing any of the above compositions and extracting the hydrolyzate with an organic solvent; a composition containing an oxidizable substance which comprises one member selected from among food and drink, medicines, quasi drugs and feed; and a composition wherein the oxidizable substance is one member selected from among oils, fat, natural coloring matters and vitamins.

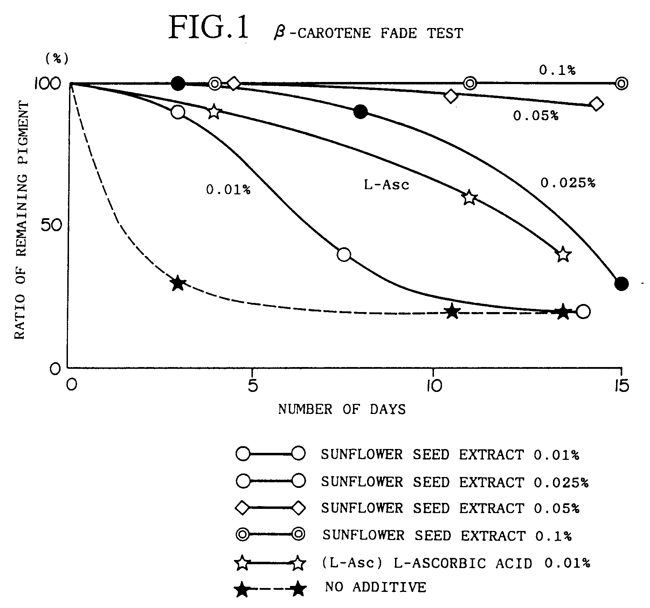

FIG.1 $\beta$-CAROTENE FADE TEST

The present invention relates to a composition obtained by extracting sunflower seeds or the dregs of sunflower seeds, and to a composition comprising the aforementioned composition and a substance receptive to oxidation.

Background

Such items as food and beverage products, medicinal products, topical applications (i.e., cosmetics), animal feed and the like, which contain fats and oils, particularly those containing unsaturated bonds, there is a tendency for acidification to occur during the period of preservation. Accordingly, some kind of antioxidant is utilized in such products. Synthetic compounds of, for example, di-n-butyl hydroxy toluene (BHT), butyl hydroxy anisole (BHA) and the like have been widely used as oxidant inhibitors. Recently, however, the use of such compounds has been reexamined in light of doubts raised concerning their carcinogenic effects. As natural products have become increasingly attractive due to these various reasons, the demand for natural source tocopherol (vitamin E) type products has been increasing. On the other hand, a large number of the other naturally derived, antioxidant substances such as sugar, amino acids and the derivatives of these two, as well as spices and the like are known to be natural sources of antioxidation components. As water soluble antioxidant substances, tea leaf extract (catechin), gallic acid, ascorbic acid or the like have been known. Recent developments of a rice sugar derived, water soluble antioxidant substance have been limited to Japanese Patent Application, First Publication Sho 62-241985. Furthermore, although screening reports on antioxidant components derived from the seeds of the sunflower have been made (18th Oil Chemistry Debate Preliminary Publication, p. 74, 1979, Takagi, Iida, et al.; Agricultural Biological Chemistry, 47(9), 2001-2007, 1983, Jong-Ho Lee, et al.), both extraction methods utilize organic solvents of a mix of hexane, ethyl ether, ethanol or chloroform methanol. It was reported that this extracted component showed either no, or only considerably weak activity. Additionally, an oxidant inhibitor obtained by culturing Aspergillus fungi on a culture medium derived from a plant seed such as the extracted dregs of sunflower seeds or bissus is disclosed in Japanese Patent Application, First Publication Sho 57-147582.

Natural coloring, in particular carotenoid pigments such as $\beta$-carotene, crocin (gardenia pigment), capsanthin (paprika pigment), and anthocyanin pigments such as the pigments of red cabbage, and purple maize, undergo oxidation and fade quickly in light or air. For this reason, vitamin C (L-ascorbic acid) has been principally used in the past as an oxidation inhibitor to prevent discoloration. Other compounds also used for this purpose include vitamin E ($\alpha$-tocopherol acid), rutin, etc.

It is known that vitamins, in particular vitamin C (L-ascorbic acid) and its derivatives, are unstable, and easily undergo oxidative decomposition by light or the oxygen in air. Due to their extreme instability, in particular in the neutral pH range, the development of a vitamin C derivative having superior stability has been frequently attempted. However, a compound which is satisfactory with respect to stability, physiological side effects, safety, cost, etc. has not been developed until now.

Recently, an ascorbic acid glucoside which is an ascorbic acid derivative using a sugar transferase, having superior stability was developed (Fragrance Journal, 1990-5).

A Brief Description of the Figures

FIG.1 shows a discoloration test for $\beta$-carotene.
FIG.2 shows the results of a discoloration test for Spirillium pigment.
FIG. 3 shows a comparison of the efficacy of conventional antioxidation components in retarding L-ascorbic acid break down.

Field of the Invention

Presently, among those products out on the market as oxidation inhibitors, oil soluble compounds such as tocopherol, etc., are the most popular. However, the range of usage of oxidation inhibitors has expanded in such areas as the processed marine products and stockbreeding food product industries. This, combined with the wish for an inhibitor against discoloration, has led to a strong desire for a water soluble antioxidant. Additionally, when oil soluble tocopherol or the like is used as an oxidation inhibitor in dried sardines, a disadvantage arise, which tocopherol flows out along with the fish oils that are floating in the boiling liquid.

Furthermore, neither is the efficacy of vitamin C as an inhibitor to oxidation sufficient. Rather, the result was obtained wherein, with the addition of L-ascorbic acid, the discoloration of such natural coloring as phycocyanin blue (a blue pigment obtained from Spirillium which is a type of blue-green algae), was accelerated.

3

Furthermore, because $\alpha$-tocopherol acid, rutin, and the like are difficult to dissolve in water, their application is naturally limited.

Many reports, including clinical examples, are available concerning the safety and physiological side effects of L-ascorbic acid. In contrast, however, the same cannot be proved of ascorbic acid derivatives, and a great deal of time and testing on their effects is still necessary.

Accordingly, it is the object of the present invention to provide a water and oil soluble antioxidant composition which is highly safe, and the efficacy of which is superior to that of L-ascorbic acid, tocopherol, or the like.

Methods of the Present Invention

Based on these circumstances, the search for a highly safe, water soluble, oil soluble antioxidant compound was completed with the discovery of the presence of an active water soluble antioxidation component in the aqueous or aqueous alcohol extract of sunflower seeds and their dregs, and, further, with the discovery of the presence of an active oil soluble antioxidant component in the hydrolyzed organic extract of the aforementioned active water soluble antioxidation component.

In other words, the present invention provides a composition comprised of a composition which includes a substance prone to oxidation and at least one of either the water soluble antioxidant substance, obtained by a water or aqueous alcohol extraction of sunflower seeds or their dregs, and the oil soluble antioxidant compound, obtained by an organic extraction of the hydrolyzed aforementioned water soluble substance.

(Structure)

A detailed explanation of the present invention follows.

The sunflower seeds utilized in the present invention are from the sunflower achene of the chrysanthemum family (Japanese name: Himawari, Chinese name: Xiàngrìkuí, scientific name: Helianthus annuus L.), and are principally utilized in sunflower oil and in pet feed. Furthermore, although the dregs following extraction are normally used as fodder for domestic animals, these too can be appropriately used as the material of the present invention. In the examination conducted by the inventors of the present invention, the seed husks were found to contain a greater amount of the active component than did the inside contents of the seeds. In the present invention, material from which the oil and fat components have been removed by means of hexane or some other organic solvent following extraction is, of course, preferable. In order to obtain the water soluble antioxidation composition according to the present invention, sunflower seeds are ground in a grinder or the like until they are of an appropriate granular size. Provided that the husks have been already crushed in the extracted dregs, the substance may be used in that form. With respect to these, in general, approximately 5~20 times more water or aqueous alcohol may be added. When extracting with water, in general it is permissible to heat by 50~100°C or to autoclave (120°C at 1 atmospheric pressure). Furthermore, when extracting with aqueous alcohol, a 90% or less by volume, and preferably a 75% or less by volume, alcohol solution is utilized. Although the extraction temperature can be changed in response to the alcohol concentration, when the alcohol concentration is above 20 vol%, and particularly when above 30 vol%, an extraction temperature of from room temperature to 70°C, preferably around 50°C, is utilized. The water soluble antioxidation composition of the present invention can be obtained either in a dried and powdered form following vacuum concentration, or can be obtained in the form of a paste or aqueous ethanol solution.

On the other hand, in order to obtain the oil soluble antioxidant composition, the above extracted liquid is first vacuum concentrated and, once the quantity of the solid component occupies approximately 5~50% by weight, and preferably 10~30% by weight, hydrolysis is performed, or the dried, powdered material is dissolved in water to obtain the above concentration and hydrolysis is carried out.

Although either the enzyme method or an acid-alkali method can be used as the method of hydrolysis, from an economic standpoint, the acid-alkali method is preferable.

As an acid used in the acid-alkali method, 0.1 N ~ 5 N solution of hydrochloric acid and sulfuric acid or a 0.5 N ~ 5 N solution of phosphoric acid is preferable. 0.1 N ~ 5 N solution of sodium hydroxide or potassium hydroxide is preferable as the alkali.

Furthermore, for both the processing temperature and processing time, the conditions necessary for carrying out hydrolysis can be optionally set in the range of 30°~100°C and from 10 minutes to 5 hours.

In order to complete hydrolysis during the extraction process, the above acid or alkali is used and extraction is performed under the same conditions.

Following cooling to room temperature of the thus processed solution, the oil soluble component can be extracted using an organic solvent from the acid treated solution as is, or can be extracted following neutralization of the solution with sodium hydroxide or potassium hydroxide. An alkali treated solution is instantly neutralized and can be extracted in the same manner.

As the organic solvent, ethyl acetate, chloroform, di-ethyl ether, di-chloro methane, ethanol, propanol, isopropanol, butanol or the like may be appropriately used. However, for extraction under the above conditions, water insoluble ethyl acetate, di-ethyl ether, chloroform or the like is preferable.

The oil soluble antioxidant composition of the present invention can, following vacuum concentration, be obtained as a dried powder, or in the form of a paste or ethanol solution.

The antioxidant composition of the present invention may be added directly to the substances susceptible to oxidation, for example, fats and oils, natural coloring, vitamins, etc., or may be added to a composition which includes the substance susceptible to oxidation, for example food and beverage product, medical products, topical applications (i.e., cosmetics), animal feed and the like. In this case, the additive quantity is normally 0.0005~1% by weight, and preferably 0.0025~0.1% by weight.

As the fat and oils, animal fats such as lard and tallow, vegetable oils such as soy or corn oil, and fish oils such as sardine oil are available. As the natural coloring, any type is appropriate, with, for example, carotenoid pigment, anthocyanin pigment, anthraquinone pigment, chalcone pigment, and Spirillium blue pigment being available. As the vitamin, for example, vitamin C, vitamin A and vitamin B are available.

The water soluble antioxidant composition of the present invention can in particular be used to prevent oxidation in, for example, emulsified fat and oil products such as butter, margarine, salad dressing, mayonnaise or shorting, meat products such as ham or sausage, marine products such as fish sausage, boiled fish paste, or chikuwa (a variety of fish sausage), and especially in dried products such as dried sardines, dried whole fish, or salted fish. Furthermore, the water soluble antioxidant composition of the present invention may also be used to prevent browning and off-flavoring, to prevent the decomposition of canned fish or meat,and canned foods, to prevent the acidification of fruit juice and beverages, to prevent fading and, in particular, to prevent the degradation of $\beta$-carotene.

The oil soluble antioxidant of the present invention can be utilized by being added to fats and oils themselves, or by being added to food and beverage products, medicinal products, topical applications, animal feed or the like, which contain fats and oils. In this case, the quantity used is usually 0.0005~1% by weight, and preferably, 0.0025~0.1% by weight.

The measurement of the antioxidant ability in water was taken by measuring the peroxide number according to the ferric rhodan method based on the method of Mitsuda, et al. (Nutrition and Food, Vol. 19, No. 3, p. 60). Comparative activity was shown as induction time, indicating the number of days until the 500nm O.D. value exceeded 0.3.

Measurements of antioxidation ability in an emulsion were carried out as follows. The water soluble antioxidant composition was added to lard as an aqueous solution, while water was added to the oil soluble antioxidant composition after it had been mixed in lard. Monoglyceride was added to each of these mixtures which were then respectively emulsified in a homogenizer. In order to prevent paralleling off of emulsification, the mixtures were maintained in a constant temperature device and the peroxide value was measured at timed intervals. The POV measurements were carried out based on the improved Lea method recorded in "Handbook of Food Analysis", p. 155 (Kenpaku Corp., published on Nov. 20, 1975).

Effects of the Present Invention

The antioxidant composition of the present invention is derived from sunflower seeds, a natural substance, is highly safe, and possesses extremely superior antioxidant activities.

Example

Next, a explanation of the present invention will be offered through examples and tests. However, these are not intended in any way to limit the scope of the present invention.

Example 1

After grinding 400 grams of sunflower seeds in a coffee mill, 2 liters of tap water was added and then autoclaved (120°C) for 30 minutes. After filtering with a Buchner funnel, 2 liters tap water was added again to the remaining dregs and autoclaving was again carried out for 30 minutes. After filtering with a Buchner funnel, the obtained extracted liquid was mixed with the extracted liquid earlier obtained and vacuum concentration was performed in an evaporator. After concentration to about 200 milliliters, freeze drying was performed, obtaining 38.6 grams of a water soluble, antioxidant composition.

Example 2

420 grams of sunflower seeds were ground in a coffee mill. To this was added 2 liters of a 50 vol% aqueous methanol solution. Extraction for 5 hours was carried out while heating to 50°C. The liquid extracted following filtering with a Buchner filter was vacuum concentrated in an evaporator. After concentration to approximately 200 milliliters, freeze drying was carried out. 23.2 grams of a water soluble antioxidant composition were obtained as a result.

Example 3

400 grams of sunflower seeds were ground in a coffee mill. To this was added 2 liters of a 50 vol% aqueous ethanol solution. Following extraction for 3 days at room temperature, the solution was filtered through a Buchner funnel. The obtained extracted liquid was then vacuum concentrated to approximately 10 milliliters in an evaporator, and following this, was freeze dried. 10.2 grams of a water soluble, antioxidant composition was obtained.

Example 4

2 liters of a 50 vol% aqueous methanol solution were added to 332 grams of dregs extracted from sunflower seeds. After soaking during a day at room temperature, the solution was heated to 50°C and extracted for 3 hours. After filtering the obtained extract through a Buchner funnel, vacuum concentration was carried out in an evaporator to approximately 100 milliliters and then freeze drying was performed. 16.1 grams of a water soluble, antioxidant composition resulted.

Experiment 1 Test of Antioxidation Ability in Water Phase

1.026 milliliters of a linoleic acid diluted to 2.51% by a 99.5% ethanol solution, and 2 milliliters of a 0.05 M phosphate buffer (pH 7.0) were added to a 25 milliliter brown screw tube. The water soluble antioxidant substances obtained in examples 1 through 4 were added so as to be in a ratio of 10~100 ppm with respect to the test liquid. Following this, water was added to bring the complete volume up to 5 milliliters. Furthermore, for comparison, dl - $\alpha$-tocopherol (reagent produced by Wako Pure Chemical Industries, Ltd.), (+)catechin, quercetin dihydrate (both reagents produced by Carl Roth, Inc.) were added in the same manner. This screw tube was maintained in the dark at 50°C.

Next, timed samplings of 0.1 milliliters of the composition solution were made and diluted with 4.8 milliliters of a 75 vol% ethanol solution. To this was added 0.05 milliliters of a 30% aqueous ammonium thiocyanate solution and 0.05 milliliters of a 3.5% ferrous chloride hydrocloric solution, and, after exactly 3 minutes, measurements of the absorbance at 500nm were taken. A standard for the acidification of the linoleic acid in this method was taken as starting from an O.D. value of 0.3 or greater. The number of days until this criteria was reached was defined as the induction time and comparisons were made of the oxidation activity.

Experimental results are shown in Table 1. These results indicate that all of the water soluble antioxidant compositions obtained in examples 1-4 possess a high degree of activity as compared to tocopherol or other natural antioxidant components.

Table 1

| Antioxidant Composition | | Induction Time | | |
|---|---|---|---|---|
| | Quantity Added | 10ppm | 500ppm | 100ppm |
| 1.Antioxidant Composition of Example 1 | | 6 days | 10 days | 20 days< |
| 2.Antioxidant Composition of Example 2 | | 12 days | 20 days< | 20 days< |
| 3.Antioxidant Composition of Example 3 | | 10 days | 20 days< | 20 days< |
| 4.Antioxidant Composition of Example 4 | | 15 days | 20 days< | 20 days< |
| Comparatives | | | | |
| 6.dl-α-tocopherol | | 2 days | 3 days | 2 days |
| 7.(+) catechin | | 4 days | 3 days | 4 days |
| 8.quercetin dihydrate | | 2 days | 4 days | 8 days |
| no additives | | 1.5 days | | |

Experiment 2 Test of Antioxidation Ability in Emulsion Phase

In a base composition of 90 grams of lard, 10 grams water, and 0.5 grams of monoglyceride, 0.05 grams and 0.1 grams of the antioxidant of the present invention obtained in the second example were dissolved in water. Furthermore for the purposes of comparison, 0.03 grams of a tocopherol mix (produced by Eisai Corp.) was mixed into lard and each component was stirred in a homogenizer for approximately 20 seconds. The obtained emulsion was maintained in a constant temperature device at 20° C, timed 1 gram sampling was performed, and POV measurements were carried out according to the conventional method. The results are shown in Table 2. These results clearly show the superior effects of the antioxidant composition of the present invention.

Table 2

| Sampling Prod. Test Item | no additives | Product of the Present Invention | | M-Toc. |
|---|---|---|---|---|
| | | 0.05% | 0.1% | 0.03% |
| week 0 | 2.3 | 2.1 | 2.2 | 2.0 |
| week 2 | 3.9 | 2.8 | 2.5 | 3.3 |
| week 5 | 7.1 | 3.8 | 2.8 | 4.4 |
| week 8 | 59.3 | 5.5 | 3.2 | 6.4 |
| M-Tox. is an abbreviation indicating Tocopherol mix | | | | |

Example 5

500 grams of sunflower seeds were ground in a coffee mill and to this was added 3500 milliliters of tap water. Extraction was carried out at 80-85° C for 5 hours. After filtering with a Buchner filter, concentration was carried out in an evaporator. Following this, freeze drying was performed. 43 grams of a water soluble, antioxidant composition was obtained.

Experiment 3 Antifade efficacy of β-carotene derived coloring

A 0.025% solution was made from water soluble, powdered β-carotene (1.5%) (produced by Sankyo, Inc.). The water soluble, antioxidant composition obtained in the fifth example of the present invention was added so as to form solutions of 0.01%, 0.025%, 0.05% and 0.1%. Furthermore, for the purpose of comparison, a solution to which 0.1% ascorbic acid had been added was prepared. 100 milliliters of each of these solutions respectively was contained within transparent glass vessels and maintained for 15 days under 3000 lux transparent light, and color fade tests were carried out. As is shown in FIG.1, the test solution having no additives had completely faded after 5 days. However, fading for the test solution with the 0.01% additive did not exceed 20%. Furthermore, the test solution having a 0.1% additive of L-ascorbic acid had, by the 13th day, experienced 50% fading. However, fading did not exceed 10% even past the 15th day for those solutions having 0.05% additive or more.

Experiment 4 Antifade efficacy of Spirillium blue derived coloring

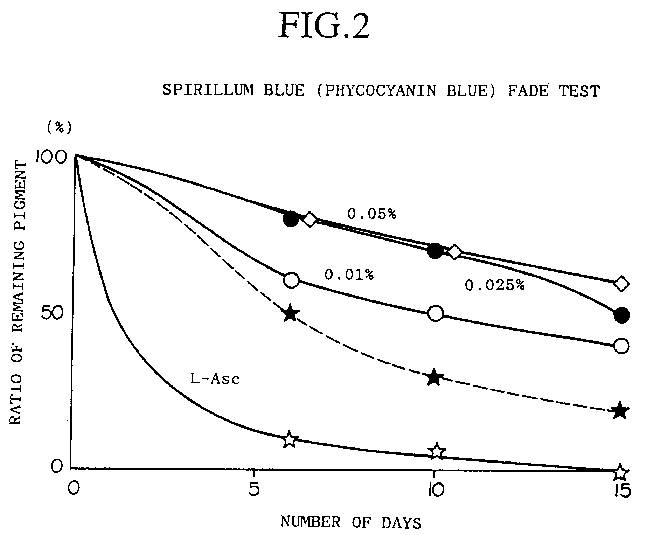

With the exception of changing to a 0.1% solution of phycocyanin blue A (produced by DaiNihon Chemical Ink Industry), the experimental materials were added by a method identical to that of experiment 3. The results are shown in FIG. 2. The substance having no additive experienced approximately 50% fading after 6 days. However, with a 0.025% additive, fading was held down to approximately 20%. These effects did not change even when the concentration of the additive was increased. On the other hand, the additive of L-ascorbic acid faded quickly accompanying sedimentation.

Example 6

3000 milliliters of a 50 vol% aqueous ethanol solution was added to 500 grams of the dregs of sunflower seeds and extracted for 5 hours. After filtering with a Buchner filter, the mixture was concentrated in an evaporator. Following this, freeze drying was carried out and 55 grams of a water soluble, antioxidant composition was obtained.

Experiment 4 Antifade efficacy of carotene derived coloring on salted red salmon

Salted red salmon was produced by thawing frozen chopped red salmon, soaking it for approximately 8 hours in a 10% salt water solution, draining and then maintaining it.

To this was added 0.03%, 0.1% and 0.2% of the water soluble antioxidant composition obtained in example 6, and the mixtures were maintained in a refrigerator unit.

The results revealed that the area of the blood red part of the meat which had received no additives had by the second day of preservation turned white. But by the sixth day of preservation, the red color remained strong in the area of the meat to which 0.1% and 0.2% additives had been made.

Acetone extraction was carried out and measurements were made on the total carotenoid content of chopped meat which had been maintained for eight days. As is shown in the table below, the total carotenoid content increased in response to the quantity of the antifade compound of the present invention, showing that there was an effect upon the suppression of the breakdown of carotenoids.

Table 3

| Test Area | Total Carotenoid Content mg/100g) |
|---|---|
| area with no additive | 1.03 |
| area with 0.03% additive | 1.28 |
| area with 0.1% additive | 1.35 |
| area with 0.2% additive | 1.54 |
| prior with reservation | 1.61 |

Example 7

8

500 grams of sunflower seeds were ground in a coffee mill. 5000 milliliters of tap water were added, and a 3 hour extraction was carried out at 80~85° C. After filtering through a Buchner filter the mixture was concentrated in an evaporator, and a second precipitate was centrifuged (5000 G × 10 minutes) in a cooling centrifuge. Following this, freeze drying was carried out. 32 grams of a water soluble antioxidant composition were obtained.

Experiment 5

100 milliliters of a 0.1% by weight solution (0.1 M acetate buffer solution, pH 5.3) of L-ascorbic acid was prepared. The water soluble antioxidant composition obtained in experiment 7 was added in amounts to make a 0 ppm, 20 ppm and 100 ppm solution. These were placed in transparent glass tubes indoors and the quantity of L-ascorbic acid was periodically determined. The results are shown in Table 4. The values show the quantity of L-ascorbic acid remaining (%) with respect to the original quantity.

Table 4

| Item | % Ratio of L-ascorbic acid Remaining | | |
|---|---|---|---|
| Compound of this Invention | At Time of Preparation | After One Day | After Three Days |
| 0 ppm | 100 | 52 | 14 |
| 20 ppm | 100 | 95 | 74 |
| 100 ppm | 100 | 100 | 85 |

The suppressive effect which an addition of 20 ppm of the compound of the present invention has on the breakdown of L-ascorbic acid is shown by the results in Table 4.

Comparative Test 1 Comparison to Conventional Anti-oxidation Components

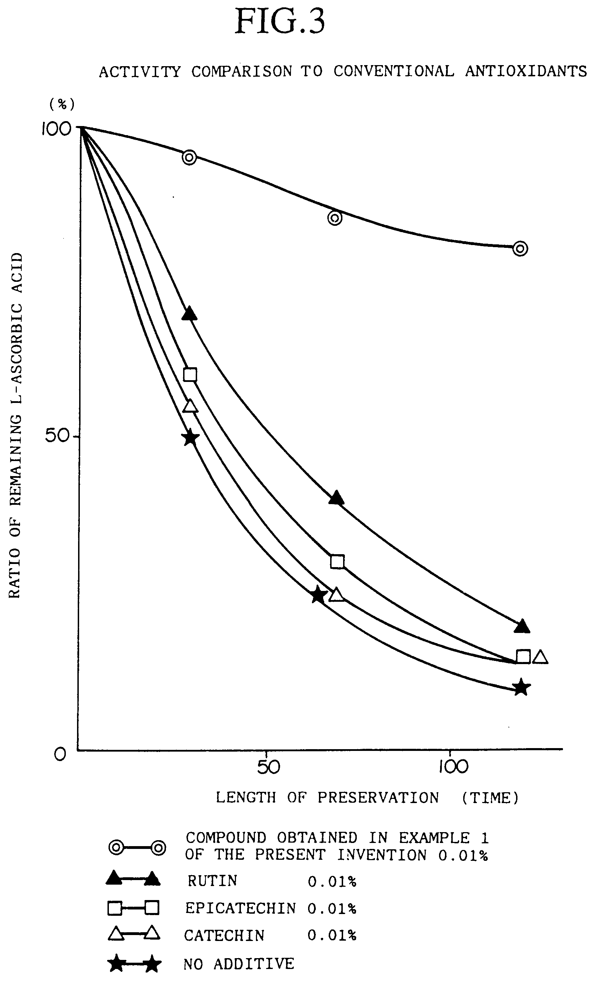

The stabilizing effect of the compound of the present invention on L-ascorbic acid was compared to that of other antioxidation components. As is shown in FIG. 3, in comparative testing of the conventional antioxidants rutin, epicatechin and catechin, no effect could be determined for any of these antioxidant components at an additive concentration of 100 ppm in a buffer solution of pH 5.3.

Example 8

400 grams of sunflower seeds were ground in a coffee mill. 4000 milliliters of tap water was added, and a 3 hour extraction was carried out at 80~85°C. After filtering through a Buchner filter, concentration to 200 ml (solid fraction 17%) was carried out in an evaporator. 20 milliliters of concentrated hydrochloric acid solution was added and a one hour hydrolysis at 90°C was carried out. Following this, the solution was cooled in ice and extracted three times with 100 milliliters of ethyl acetate. Following dehydration of the obtained organic layer of ethyl acetate with anhydrous sodium sulfate, the solvent was removed and 4.8 grams of the yellowish-brown, oil soluble antioxidant composition of the present invention was obtained.

Example 9

5000 milliliters of a 70% ethanol solution was added to 500 grams of sunflower seed shells and a 5 hour extraction was carried out at 50°C. Following filtering through a Buchner filter, the extracted solution was concentrated in an evaporator. Freeze drying was then carried out and 32.5 grams of a water soluble, antioxidant composition was obtained. 150 milliliters of 0.5 N sulfuric acid was added to 15 grams of the obtained substance, and hydrolysis was carried out at 90°C for 30 minutes. After cooling, extraction was carried out three times using 100 milliliters of diethyl ether. 2.2 grams of an oil soluble antioxidant composition were obtained by the same process as in the preceding eighth example.

Experiment 6 Antioxidation Test in Lard

10 grams of lard to which no antioxidant composition had been added was places in a 20 milliliters brown screw tube. To this was added an ethanol solution of the antioxidant composition obtained in the example 8 of the present invention so as to make a 100ppm solution. Following treatment at 180°C for 15 minutes, the solution was maintained at a constant temperature of 50°C, and the peroxide value was periodically measured. Furthermore, as a comparison, measurements were made for the case where 200 ppm of DL-$\alpha$-tocopherol (hereafter abbreviated as Toc.) was added. As indicated in Table 5, the antioxidant composition of the present invention showed superior antioxidation effects in lard. In contrast, on this point, the antioxidant component prior to hydrolysis showed no effect.

Additionally, measurements of the peroxide value (hereafter abbreviated as POV) were carried out according to the Lea method recorded in paragraph 155 of "Handbook of Food Analysis" (published by Kenpaku Corp., November 20 1977).

Table 5

| Quantity of Specimen Added | POV (meq/kg | | |
|---|---|---|---|
| | 6 days | 12 days | 16 days |
| Compound of Present Invention 100ppm | 11.8 | 7.8 | 10.6 |
| Toc. 200ppm | 16.4 | 21.8 | 36.4 |
| Pre-hydrolized product 100ppm | 29.8 | 45.4 | 72.8 |
| No Additive | 33.8 | 50.4 | 92.6 |

Corresponding Example 1

The following skin lotion compounds were manufactured.

| Compound | Weight (%) |
|---|---|
| olive oil | 15.0 |
| myristic acid isopropyl | 5.0 |
| poly(oxyethylene) nonylphenyl ether | 0.5 |
| propylene glycol | 3.0 |
| glycerol | 5.0 |
| **chiparaben** | 0.1 |
| ethanol | 7.0 |
| compound of the experiment 9 of the present invention | 0.02 |
| purified water | added so as to bring the total weight to 100 |

The samples, which were maintained for 4 months at 40°C, had no offensive smell and showed almost no change from their condition soon after the preparation. In contrast to this, a color change and an offensive odor were noted in the samples to which the antioxidant of the present invention had not been added.

**Claims**

1. An antioxidant compound obtained through the extraction of sunflower seeds or the dregs of sunflower seeds by an aqueous alcohol solution.

2. An antioxidant compound according to claim 1 wherein the alcohol of said aqueous alcohol is methanol and/or ethanol.

3. An antioxidant compound according to one of claims 1 or 2 wherein the alcohol content of said aqueous alcohol is 90 vol% or less.

4. An antioxidant compound according to one of claims 1 or 2 wherein the alcohol content of said

10

aqueous alcohol is 75 vol% or less.

5. An antioxidant compound obtained by the hydrolysis of said antioxidant compound of claim 1, and by extraction with an organic solvent.

6. A composition comprising a compound which includes at least one of said antioxidant compound according to claim 1 and said antioxidant compound according to claim 5 and a compound prone to oxidation or a composition including said compound prone to oxidation.

7. A composition according to claim 6 wherein said composition includes a compound prone to oxidation and is one selected from the groups consisting of food and beverage products, medicinal products, topical applications and animal feed products.

8. A composition according to one of claims 6 and 7 wherein the compound prone to oxidation is one selected from the groups consisting of fats and oils, natural coloring and vitamins.

## FIG.1 β-CAROTENE FADE TEST

# FIG.2

SPIRILLUM BLUE (PHYCOCYANIN BLUE) FADE TEST

# FIG.3

ACTIVITY COMPARISON TO CONVENTIONAL ANTIOXIDANTS

COMPOUND OBTAINED IN EXAMPLE 1 OF THE PRESENT INVENTION 0.01%

RUTIN 0.01%

EPICATECHIN 0.01%

CATECHIN 0.01%

NO ADDITIVE

# INTERNATIONAL SEARCH REPORT

International Application No PCT/JP91/00201

## I. CLASSIFICATION OF SUBJECT MATTER (if several classification symbols apply, indicate all) 6

According to International Patent Classification (IPC) or to both National Classification and IPC

Int. Cl$^5$  C09K15/34

## II. FIELDS SEARCHED

### Minimum Documentation Searched 7

| Classification System | Classification Symbols |
|---|---|
| IPC | C09K15/34, A23L3/3472 |

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 57-147582 (Higashimaru Shoyu K.K.), September 11, 1982 (11. 09. 82), Claims 1 to 3, lines 2 to 14, lower right column, page 2, line 9, lower left column to line 14, lower right column, page 3 (Family: none) | 1-8 |
| A | JP, A, 2-4899 (Société des Produits Nestle S.A.), January 9, 1990 (09. 01. 90), Line 8, upper left column to line 1, upper right column, page 2 & EP, A, 326829 | 1-8 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| May 13, 1991 (13. 05. 91) | May 27, 1991 (27. 05. 91) |

| International Searching Authority | Signature of Authorized Officer |
|---|---|
| Japanese Patent Office | |